Europäisches Patentamt

European Patent Office

Office européen des brevets

(19)

(11)     EP 0 733 903 B1

(12)     **FASCICULE DE BREVET EUROPEEN**

(45) Date de publication et mention
de la délivrance du brevet:
**27.02.2002   Bulletin 2002/09**

(51) Int Cl.⁷: **G01N 33/74, C07K 16/26**

(21) Numéro de dépôt: **96430001.6**

(22) Date de dépôt: **22.03.1996**

(54) **Procédé de dosage immunologique du cortisol, notamment urinaire, et réactifs utilisés**

Verfahren und Reagenzien zum immunologischen Nachweis von Cortisol im Urin

Immunoassay for the detection of urinary cortisol and reagents therefor

(84) Etats contractants désignés:
**AT BE CH DE ES FI FR GB IE IT LI MC NL PT**

(30) Priorité: **24.03.1995  FR 9503749**

(43) Date de publication de la demande:
**25.09.1996   Bulletin 1996/39**

(73) Titulaires:
• **Société Anonyme dite: IMMUNOTECH S.A.**
**F-13276 Marseille Cédex 9 (FR)**
• **INSTITUT NATIONAL DE LA SANTE ET DE LA**
**RECHERCHE MEDICALE (INSERM)**
**75654 Paris Cédex 13 (FR)**

(72) Inventeurs:
• **Lupi-Chen, Nina**
**La Valentine 13011, Marseille (FR)**
• **Mappus, Elisabeth**
**69110 Ste Foy les Lyon (FR)**
• **Fournier, Catherine**
**69100 Villeurbanne (FR)**

• **Barrande, Christophe**
**13007 Marseille (FR)**
• **Portuesi, Sandrine**
**13007 Marseille (FR)**
• **Cuilleron, Yves-Claude**
**69110 Ste Foy les Lyon (FR)**

(74) Mandataire: **Rinuy, Santarelli**
**14, avenue de la Grande Armée**
**75017 Paris (FR)**

(56) Documents cités:
**DE-A- 2 720 809          FR-A- 2 537 586**
**US-A- 4 339 390**

• **TRAC, TRENDS IN ANALYTICAL CHEMISTRY,**
**vol. 8, no. 2, Février 1989, AMSTERDAM NL,**
**pages 72-75, XP000035746 R.HAMPL ET AL.:**
**"RECENT ADVANCES IN IMMUNOASSAY OF**
**STEROID HORMONES"**

## Description

La présente invention concerne un nouveau procédé de dosage immunologique du cortisol sans extraction ni chromatographie, basé sur les propriétés surprenantes de certains anticorps dirigés contre le cortisol.

L'invention comprend notamment les anticorps eux-mêmes, un procédé pour les obtenir, des procédés de dosage du cortisol, basés sur ces anticorps et des trousses de réactifs permettant la mise en oeuvre de ces procédés.

Le cortisol, principale hormone glucocorticoïde, est synthétisé dans les zones fasciculées et réticulées de la corticosurrénale. Il dérive du cholestérol sous l'action de plusieurs enzymes. Son taux de sécrétion est sous la dépendance d'une régulation hypothalamo-hypophysaire. Le CRF (Corticotropin Releasing Factor) active l'antéhypophyse qui produit l'ACTH, celle-ci stimule à son tour le cortex surrénalien qui synthétise des corticoïdes dont 90% de cortisol. Dans le plasma, la majorité du cortisol circule sous forme liée aux protéines plasmatiques : principalement à la CBG corticosteroid binding globulin, à l'albumine et à la TEBG testostérone estradiol binding globulin; une très faible fraction existe sous forme libre.

L'exploration fonctionnelle de l'axe hypothalamus-hypophyse-corticosurrénale est largement basée sur le dosage du cortisol. Sa mesure dans le sérum est utilisée essentiellement au cours des épreuves dynamiques. Le cortisol libre urinaire, enfin, a une bonne corrélation avec le taux de production de l'hormone, et reste le meilleur indice hormonal dans les cas d'hypercorticisme.

Le dosage direct du cortisol dans le sérum est facilité par son taux généralement élevé par rapport à l'ensemble des autres stéroïdes, à l'exception de la déhydro-épiandrostérone (DHEA) sulfate qui appartient à la famille des androgènes de structure très différente et est peu reconnue par les anticorps anti-cortisol. Toutes les trousses commercialisées de type radio-immunologique ou immunoenzymologique proposent des techniques de mesure directe du cortisol dans les sérums. Elles sont souvent de qualité souhaitée (voir Tableau 1 ci-après).

En revanche, le dosage direct du cortisol urinaire présente une plus grande difficulté, dûe essentiellement à l'accumulation de métabolites, de structure et de concentration souvent mal connues. Jusqu'à présent, les procédés de dosages du cortisol les plus fiables dans ce milieu biologique, comprennent une étape d'extraction au dichlorométhane suivie d'une chromatographie qui assure la spécificité. Ces opérations sont délicates, longues et coûteuses.

De nombreuses tentatives ont été effectuées pour engendrer des anticorps monoclonaux et polyclonaux anti-cortisol, notamment à partir d'un dérivé cortisol-21-hémisuccinate et surtout d'un dérivé cortisol-3-CMO, sans description dans de ce dernier cas de propriétés spécifiques liées à l'isomérie géométrique syn/anti du groupe CMO. De tels anticorps ne permettent pas de doser correctement le cortisol urinaire : ils surestiment les concentrations du cortisol dans les urines, en comparaison des résultats obtenus par dosage après chromatographie.

Trois fabricants : Kodak, Orion et DSL proposent des dosages directs du cortisol urinaire. Seulement, comme le montre le tableau 2 ci-après, ces procédés donnent des résultats bien différents de ceux obtenus par dosage après extraction et chromatographie de l'échantillon.

De plus, le document US-A-4 339 390 décrit un anticorps obtenu par immunisation avec l'haptène cortisol-21-acétate-3-carboxyméthyloxime couplé à de la sérum-albumine bovine (BSA) avec un taux de couplage de 22. Le rapport des affinités du-dit anticorps pour les isomères syn et anti d'un dérivé du cortisol, le cortisol-21-acétate-3-iminoxyacétyle tyrosine méthyle ester, est supérieur à 1 en faveur de l'isomère syn. Cependant, l'anticorps décrit dans ce document ne reconnaît pas en milieu urinaire les isomères syn et anti d'un autre dérivé du cortisol, le cortisol-3-carboxyméthyloxime-histamine, avec un rapport des affinités qui est également égal ou supérieur à 1 en faveur de l'isomère syn. De plus, cet anticorps a été obtenu par immunisation avec l'haptène cortisol-21-acétate-3-carboxyméthyloxime, et non pas du cortisol-3-carboxyméthyloxime couplé à du BSA.

Il serait donc souhaitable de disposer d'une méthode de mesure directe du cortisol urinaire rapide et donnant un résultat proche de celui obtenu par chromatographie, technique trop lourde pour être utilisée en routine.

Or, la demanderesse a préparé certains anticorps et découvert que ceux-ci, qu'ils soient polyclonaux ou monoclonaux, présentent une spécificité particulière qui permet un dosage direct non seulement du cortisol dans le sérum et dans d'autres fluides biologiques (salive, liquide céphalo-rachidien, surnageants de culture) mais aussi du cortisol dans l'urine.

Ces anticorps selon l'invention peuvent être notamment obtenus chez des animaux immunisés avec un nouvel immunogène constitué du haptène : le cortisol-3-carboxyméthyloxime (isomère syn) substantiellement pur, couplé à une macromolécule, notamment par sa fonction carboxylique.

C'est pourquoi la présente invention a pour objet un nouvel anticorps polyclonal ou monoclonal, reconnaissant spécifiquement le cortisol en milieu urinaire et dont le rapport des affinités (CI50) de l'anticorps pour les dérivés cortisol-3-carboxyméthyloxime (syn)-histamine et cortisol-3-carboxyméthyloxime (anti)-histamine, est supérieur ou égal à 1, préférentiellement supérieur à 1,5, en faveur de l'isomère syn, tel qu'obtenu par utilisation d'un immunogène comprenant à titre de haptène le cortisol-3-carboxyméthyloxime (isomère syn) substantiellement pur. Le cortisol-3-carboxyméthyloxime sera parfois appelé ci-après cortisol-3-CMO.

Par "macromolécule", l'on entend notamment une protéine ou un polysaccharide, de poids moléculaire suffisant pour induire la production d'anticorps chez les animaux, et de préférence la sérum-albumine bovine (SAB). Selon la plupart des auteurs, un poids moléculaire de 5000 est considéré comme suffisant.

Par "couplé", l'on entend que le cortisol-3-CMO (syn) est lié à la macromolécule notamment par une liaison covalente, par exemple ester ou amide, et de préférence une liaison amide sur les lysines d'une protéine.

Une réalisation particulière de l'invention consiste à coupler le cortisol-3-carboxyméthyloxime (syn) à la sérum-albumine bovine (SAB) avec un rapport molaire de couplage supérieur ou égal à 1 et de préférence compris entre 15 et 31. On engendre ainsi chez la souris des anticorps polyclonaux d'une grande affinité et d'une spécificité remarquable permettant de doser directement le cortisol dans les urines des patients, sans extraction, ni chromatographie, en l'espace d'une heure.

On a également fusionné des cellules de myélome de souris avec des lymphocytes de souris immunisées avec l'immunogène cortisol-3-carboxyméthyloxime (syn)-SAB pour produire des anticorps monoclonaux dont la spécificité est quasiment identique à celle des anticorps polyclonaux correspondants.

La présente invention a donc en particulier pour objet de tels anticorps monoclonaux, notamment un anticorps monoclonal secrété par l'hybridome IMMU-473, déposé le 9 Février 1995, sous le N° I 1532 à la Collection Nationale des Cultures de Microorganismes à Paris.

Les anticorps ci-dessus décrits et utilisés dans les procédés et trousses ci-après sont de préférence monoclonaux.

Le procédé d'obtention d'anticorps selon l'invention ci-dessus décrit n'est pas limitatif. Il est en particulier possible d'obtenir des anticorps selon l'invention avec des immunogènes mélangés, avec des supports qui ne sont pas macromoléculaires, par immunisation in vitro, ou même sans immunisation (Griffiths A.D. et Al.; EMBO Journal, 13, 3245-3260, 1994).

L'invention a aussi pour objet des procédés de dosage du cortisol basés sur les anticorps polyclonaux ou monoclonaux décrits ci-dessus dans diverses techniques de dosage utilisant des marqueurs de caractère radioactif (tritium ou iode radioactif par exemple), enzymatique, luminescent ou fluorescent, en phase solide ou en phase liquide. De tels marqueurs peuvent être couplés soit sur un dérivé du cortisol, soit sur un anticorps monoclonal ou polyclonal selon l'invention, pour constituer le traceur correspondant. Le cortisol tritié ou un dérivé du cortisol tritié peut constituer aussi un traceur convenable.

C'est pourquoi la présente invention a aussi pour objet un procédé de dosage immunologique du cortisol caractérisé en ce que l'on utilise un anticorps tel que défini ci-dessus, en ce que le dosage est un dosage par compétition utilisant un traceur et que le traceur est constitué du haptène cortisol-3-carboxyméthyloxime (syn) ou cortisol-3-carboxyméthyloxime (anti) couplé par sa fonction carboxylique à un marqueur qui est une molécule iodable.

Le couplage peut être direct ou réalisé par l'intermédiaire d'un bras bien connu de l'état de la technique.

Par "molécule iodable", l'on entend une molécule iodable bien connue de l'homme de l'art, par exemple l'histamine, l'histidine, la tyramine, la tyrosine ou un peptide contenant un résidu iodable tel qu'un noyau phénol ou imidazole.

Une réalisation particulière de l'invention consiste à utiliser un anticorps décrit ci-dessus fixé sur une phase solide que l'on incube en présence du cortisol à doser et d'un traceur iodé radioactif, produit par marquage à l'iode 125 d'un précurseur obtenu en greffant le cortisol-3-carboxyméthyloxime (syn) ou le cortisol-3-carboxyméthyloxime (anti) sur une molécule iodable ci-dessus.

Dans d'autres conditions préférentielles de mise en oeuvre de l'invention, le procédé ci-dessus est caractérisé en ce que, à titre de traceur, le cortisol-3-carboxyméthyloxime (syn) ou le cortisol-3-carboxyméthyloxime (anti) est couplé à l'histamine iodée radioactive.

Dans des conditions préférentielles de mise en oeuvre, le procédé ci-dessus décrit est caractérisé en ce que le traceur radioactif est iodé à l'iode 125.

Selon d'autres techniques de dosage immunologique, le cortisol à doser est mis en compétition avec un traceur qui est un conjugué constitué de cortisol-3-carboxy-méthyloxime (syn) ou cortisol-3-carboxyméthyloxime (anti) couplé à une enzyme ou à une molécule luminescente ou fluorescente.

De façon préférentielle, les anticorps polyclonaux ou monoclonaux selon l'invention sont fixés sur une phase solide, tubes, plaques de titration ou billes.

L'invention a aussi pour objet un procédé de dosage immunologique du cortisol dans lequel on utilise un dérivé du cortisol, notamment un cortisol-3-carboxyméthyloxime (syn) ou (anti), fixé sur une phase solide et un anticorps anti-cortisol tel que défini ci-dessus, marqué par une molécule radioactive, enzymatique, luminescente ou fluorescente.

Dans ce dernier procédé, on incube le cortisol à doser en présence d'un dérivé du cortisol immobilisé ci-dessus et d'un anticorps selon l'invention, marqué.

La présente invention a aussi pour objet des trousses de dosage du cortisol dans les fluides biologiques utilisant un anticorps tel que défini ci-dessus.

Un premier type de trousse de dosage direct du cortisol renferme une phase solide recouverte d'anticorps dirigés contre le cortisol-3-carboxyméthyloxime (syn) -SAB selon l'invention.

Une telle trousse renferme de préférence en outre un traceur "cortisol-3-carboxyméthyloxime (anti)" ou "cortisol-3-carboxyméthyloxime (syn)" couplé par sa fonction carboxylique soit à une enzyme, soit à une molécule fluorescente ou luminescente soit à une histamine, ou à un peptide ou à un dérivé d'acide aminé comprenant un noyau phénol ou un noyau imidazole, iodé par un atome d'iode radioactif. Une trousse encore plus complète comprend avantageusement de plus une ou de préférence deux gammes standard de cortisol,l'une pour le dosage du cortisol dans les urines,l'autre pour le dosage du cortisol dans les sérums.

Un deuxième type de trousse de dosage direct du cortisol contient un traceur "anticorps anti-cortisol" selon l'invention, marqué soit par l'iode radioactif, soit par une molécule luminescente ou fluorescente soit encore par une enzyme. Une telle trousse renferme de préférence en outre une phase solide recouverte d'antigène de cortisol et avantageusement de plus une ou de préférence deux gammes standard de cortisol,l'une pour le dosage du cortisol dans les urines, l'autre pour le dosage du cortisol dans les sérums.

L'invention a également pour objet le cortisol-3-carboxyméthyloxime (syn)-histamine ainsi que le cortisol-3-carboxyméthyloxime (anti)-histamine, sous forme substantiellement pure, qui peuvent être utilisés pour la caractérisation des anticorps et comme précurseurs de dérivés iodés radioactifs.

L'invention a encore pour objet le cortisol-3-carboxyméthyloxime (syn), sous forme substantiellement pure, le cortisol-3-carboxyméthyloxime (anti), sous forme substantiellement pure, et notamment le cortisol-3-carboxyméthyloxime (syn), qui peut être enrichi en isomère majoritaire à raison de 96 % au moins, particulièrement substantiellement libre de cortisol-3,20-di-carboxyméthyloxime, c'est à dire en renfermant moins de 1 %, et renfermant de préférence moins de 1% de produits de transformation des fonctions en position 11 et sur la chaîne latérale dihydroxycétone en position 17 (pourcentages estimés par chromatographie liquide à haute performance) et tout particulièrement le cortisol-3-carboxyméthyloxime (anti) enrichi en isomère majoritaire à raison de 99 % au moins, particulièrement substantiellement libre de cortisol-3,20-di-carboxyméthyloxime, c'est à dire en renfermant moins de 1 %, et renfermant moins de 1 % de produits de transformation des fonctions en position 11 et sur la chaîne latérale dihydroxycétone en position 17 (pourcentages estimés par chromatogra-phie liquide à haute performance).

L'invention a enfin pour objet le cortisol-3-carboxyméthyloxime (syn) substantiellement pur couplé à une macromolécule, notamment à une protéine, particulièrement à la sérum albumine bovine.

Des conditions dans lesquelles les hybridomes sécréteurs des anticorps monoclonaux selon l'invention ont été isolés seront décrites ci-après.

On trouvera également ci-après des exemples illustrant les dosages directs du cortisol dans les sérums et les urines, de type radio-compétition, à l'aide de ces anticorps monoclonaux. Ces exemples concernent :

1. La synthèse de l'haptène cortisol-3-CMO et séparation des isomères syn et anti.
2. La préparation de l'immunogène cortisol-3-CMO (syn)-sérum-albumine bovine
3. La préparation de l'immunogène cortisol-3-CMO (anti)-sérum-albumine bovine.
4. La préparation du précurseur de marquage: cortisol-3-CMO (syn)-histamine et du traceur iodé radioactif correspondant.
5. La préparation du précurseur de marquage: cortisol-3-CMO (anti)-histamine et du traceur iodé radioactif correspondant.
6. Des anticorps dirigés contre le cortisol-3-CMO (syn)-SAB.
7. Une trousse de dosage direct du cortisol dans les sérums ou plasmas.
8. Une trousse de dosage direct du cortisol dans les urines.

EXEMPLE 1 : Synthèse du cortisol-3-CMO et séparation des deux isomères syn et anti.

**Stade A : 21-acétoxy-cortisol (2)**

Le cortisol (1) (10,0 g; 27,6 mmol) est dissous dans 300 ml d'un mélange pyridine-anhydride acétique 5:1. Le mélange réactionnel est maintenu à 70°C pendant 75 minutes puis évaporé sous pression réduite. Les dernières traces d'anhydride acétique sont éliminées par addition d'éthanol et par distillation azéotropique de toluène sous pression réduite. On obtient l'acétate (2) sous forme d'un solide blanc (11,1 g; 27,4 mmol).

$R_f$ = 0,35 (chloroforme-acétate d'éthyle 1:1)

F = 220-224°C (après une recristallisation dans un mélange chloroforme-méthanol)

$^1$H-RMN ($C_5D_5N$) δ 1,41 (3H, s, 18-$CH_3$) ; 1,62 (3H, s, 19-$CH_3$); 2,12 (3H, s, 21-$COCH_3$) ; 4,7 (1H, m, 11α-H); 5,3-5,6 (4H, q: J=17,5 Hz, 21-$CH_2O$); 5,8 (1H, s, 4-H).

**Stade B : 21-acétoxy-cortisol-3-CMO (mélange syn + anti (3))**

Le 21-acétoxy-cortisol (2) (4,0 g; 9,9 mmol) obtenu au stade A est dissous dans 150 ml de pyridine anhydre. A cette solution est ajoutée goutte à goutte une solution d'hémichlorhydrate d'O-carboxyméthylhydroxylamine (1,3 g;

5,9 mmol) dans 150 ml de pyridine. Le milieu réactionnel est agité sous atmosphère d'azote 16 heures à température ambiante. La fin de la réaction est contrôlée par chromatographie sur couche mince, puis la pyridine est éliminée par évaporation sous pression réduite. Le résidu est repris par une solution aqueuse saturée d'hydrogénocarbonate de sodium qui est ensuite extraite au chloroforme pour éliminer les contaminants non acides. La phase aqueuse est acidifiée jusqu'à pH 4 par addition d'acide chlorhydrique et extraite à l'acétate d'éthyle. La phase organique contenant le stéroïde est évaporée puis séchée par distillation azéotropique de toluène, sous pression réduite.

On obtient le 21-acétoxy-cortisol-3-CMO ($\underline{3}$) (4,3 g; 9,0 mmol) contenant un mélange d'isomères syn et anti (40% et 60% respectivement, pourcentages estimés par RMN)

$R_f$ = 0,40 (syn) et 0,50 (anti) (chloroforme-acétone-acide acétique 7:2:1)

$^1$H-RMN ($C_5D_5N$) δ 1,38 (3H, s, 18-$CH_3$); 1,53 (3H, s, 19-$CH_3$) ; 2,11 (3H, s, 21-$COCH_3$) ; 4,6 (1H, m, 11α-H) ; 5,1 (2H, s, NO $CH_2CO$) ; 5,3-5,6 (4H, q: J=17,5 Hz, 21-$CH_2O$) ; 6,0-6,7 (1H, s, 4-H anti et syn).

### Stade C : cortisol-3-CMO (mélange syn + anti) ($\underline{4}$).

Le mélange d'isomères syn et anti du 21-acétoxycortisol-3-CMO ($\underline{3}$) (2,0 g; 4,2 mmol) obtenu au stade B est dissous dans 320 ml de méthanol, puis on ajoute 20 ml d'une solution aqueuse d'hydrogénocarbonate de potassium à 10%. Le mélange réactionnel est agité 16 heures sous atmosphère d'azote à température ambiante, puis évaporé sous pression réduite. Le résidu est repris par 100 ml d'eau et la solution est acidifiée jusqu'à pH 4 par addition d'acide chlorhydrique puis extraite à l'acétate d'éthyle. La phase organique contenant le stéroïde est évaporée puis séchée par distillation azéotropique de toluène, sous pression réduite.

On obtient le cortisol-3-CMO ($\underline{4}$) (1,6 g; 3,7 mmol) contenant un mélange d'isomères syn et anti (40% et 60% respectivement, pourcentages estimés par RMN)

$R_f$ = 0,19 (syn) et 0,27 (anti) (chloroforme-acétone-acide acétique 7:2:1)

### Stade D : Isomère syn ($\underline{5}$) et isomère anti ($\underline{6}$) du cortisol-3-CMO .

Les deux isomères syn et anti du dérivé cortisol-3-CMO (0,48 g; 1,1 mmol) obtenus au stade C sont séparés par chromatographie sur plaques de silice développées par un mélange chloroforme-acétone-acide acétique 7:2:1. On obtient 0,17 g (0,39 mmol) d'isomère syn ($\underline{5}$) et 0,21 g (0,48 mmol) d'isomère anti ($\underline{6}$) contenant chacun entre 5 et 10 % de l'autre isomère. Chaque isomère est ensuite obtenu à l'état pur par chromatographie liquide à haute performance (CLHP) sur colonne Waters C18; éluant : méthanol-eau-acide acétique 600:400:1; débit 8 ml/minute; $t_r$ (anti) 23 minutes, $t_r$ (syn) = 26 minutes. Le solvant est alors évaporé sous pression réduite.

**Isomère (syn) du cortisol-3-CMO ($\underline{5}$)**

$R_f$ = 0,19 (chloroforme-acétone-acide acétique 7:2:1)

F = 133-136°C (produit brut séparé par CLHP)

UV : $\lambda_{max}$ = 256 nm; $\in$= 15000 $M^{-1}cm^{-1}$

$^1$H-RMN ($CD_3OD$) δ 0,86 (3H, s, 18-$CH_3$) ; 1,37 (3H, s, 19-$CH_3$); 4,2-4,7 (4H, q: J=19 Hz, 21-$CH_2O$); 4,4 (1H, m, 11α-H) ; 4,5 (2H, s, $NOCH_2CO$); 6,4 (1H, s, 4-H).

**Isomère (anti) du cortisol-3-CMO ($\underline{6}$)**

$R_f$ = 0,27 (chloroforme-acétone-acide acétique 7:2:1)

F = 223-227°C (produit brut séparé par CLHP)

UV : $\lambda_{max}$ = 250 nm; $\in$= 23000 $M^{-1}cm^{-1}$

$^1$H-RMN ($CD_3OD$) δ 0,86 (3H, s, 18-$CH_3$); 1,34 (3H, s, 19-$CH_3$); 4,2-4,7 (4H, q: J=19 Hz, 21-$CH_2O$); 4,4 (1H, m, 11α-H); 4,5 (2H, s, $NOCH_2CO$); 5,6 (1H, s, 4-H).

EXEMPLE 2 : Immunogène isomère (syn) du cortisol-3-CMO couplé à la sérum-albumine bovine($\underline{11}$)

### Stade A : Isomère (syn) du cortisol-3-CMO activé sous forme d'ester de N-hydroxysuccinimide ($\underline{7}$)

L'isomère (syn) du cortisol-3-CMO($\underline{5}$) (0,1 g; 0,23 mmol) obtenu au stade D de l'exemple 1 est dissous dans 20 ml de THF anhydre en présence de N-hydroxysuccinimide (0,11 g; 0,95 mmol) et de dicyclohexylcarbodiimide (0,21 g; 1,02 mmol). Le mélange réactionnel est agité 4 heures à température ambiante sous atmosphère d'azote et à l'obscurité puis filtré. Le filtrat est évaporé à sec sous flux d'azote sans chauffer pour conduire à l'ester activé brut ($\underline{7}$), qui peut en général être employé tel quel dans la suite du procédé.

$R_f$ = 0,54 (acétate d'éthyle)

**Stade B : Immunogène isomère (syn) du cortisol-3-CMO couplé à la sérum-albumine bovine (11)**

La sérum-albumine bovine (200 mg; 3 μmol environ) est dissoute dans 8 ml d'une solution aqueuse d'hydrogénocarbonate de sodium 10 mM, à laquelle on ajoute 3 ml de THF goutte à goutte. L'isomère (syn) du cortisol-3-CMO activé sous forme d'ester de N-hydroxysuccinimide (7) (0,06 g; 113 μmol) obtenu au stade A est dissous dans 3 ml de THF. Cette solution est ensuite ajoutée goutte à goutte à la solution de sérum-albumine bovine. Le mélange réactionnel est agité à température ambiante pendant 16 heures, puis dialysé à +4°C contre une solution aqueuse d'hydrogénocarbonate de sodium 10 mM. Le dialysat est chromatographié sur colonne de Sépharose CL 4B (Pharmacia) équilibrée avec une solution aqueuse d'hydrogénocarbonate de sodium 10 mM (détection par absorbance en UV à 280 nm). Les fractions contenant l'immunogène sont réunies, dialysées contre de l'eau déionisée ajustée à pH 7 par addition d'hydrogénocarbonate d'ammonium et lyophilisées.

On obtient environ 250 mg de l'immunogène isomère (syn) du cortisol-3-CMO couplé à la sérum-albumine bovine (11), dont le taux de couplage est compris entre 27 et 31. Le nom du composé est abrégé en cortisol-3-CMO (syn)-SAB.

EXEMPLE 3 : Immunogène isomère (anti) du cortisol-3-CMO couplé à la sérum-albumine bovine (12).

**Stade A : Isomère (anti) du cortisol-3-CMO activé sous forme d'ester de N-hydroxysuccinimide (8)**

L'isomère anti du cortisol-3-CMO (6) obtenu au stade D de l'exemple 1 est traité comme indiqué au stade A de l'exemple 2 pour conduire à l'ester activé brut (8).
$R_f$ = 0,61 (acétate d'éthyle)

**Stade B : Immunogène isomère (anti) du cortisol-3-CMO couplé à la sérum-albumine bovine (12)**

L'isomère (anti) du cortisol-3-CMO activé sous forme d'ester de N-hydroxysuccinimide (8) obtenu au stade A est traité de la même manière que celle indiquée au stade B de l'exemple 2, pour conduire à l'immunogène attendu : cortisol-3-CMO (anti)-SAB (12), dont le taux de couplage est compris entre 27 et 31.

EXEMPLE 4 - Précurseur de marquage isomère (syn) du cortisol-3-CMO couplé à l'histamine (9) et traceur iodé radioactif correspondant.

**Stade A : Isomère (syn) du cortisol-3-CMO couplé à l'histamine (9)**

L'histamine base (0,05 g; 0,45 mmol) est dissoute dans 1 ml d'une solution aqueuse d'hydrogénocarbonate de sodium 10 mM, puis on ajoute 1 ml de THF. L'isomère (syn) du cortisol-3-CMO activé sous forme d'ester de N-hydroxysuccinimide (7) (0,03 g; 0,056 mmol) obtenu au stade A de l'exemple 2 est dissous dans 1,5 ml de THF. Cette solution est ajoutée goutte à goutte à la solution d'histamine. Le mélange réactionnel est agité à température ambiante sous atmosphère d'azote pendant 1 heure. La solution est ajustée à pH 7 par addition d'acide chlorhydrique puis évaporée à sec sous pression réduite. Le résidu est ensuite purifié par chromatographie sur plaques de silice développées dans un mélange chloroforme-méthanol-ammoniaque 60:10:2 puis par CLHP (colonne Shandon Ultrabase 5μ, μBondapak C18; éluant: méthanol-eau-ammoniaque 500 : 500 : 1; débit 1 ml/minute); $t_r$ (syn) = 25,3 minutes.

On obtient 0,02 g (0,038 mmol) de produit couplé à l'histamine (9). Le nom de ce produit est abrégé en : cortisol-3-CMO (syn)-histamine.
$R_f$ = 0,25 (chloroforme-méthanol-ammoniaque 60:10:2)
F = 126 - 131°C (produit brut séparé par CLHP)
UV : $\lambda_{max}$ = 253 nm; $\in$= 15000 $M^{-1}cm^{-1}$
$^1$H-RMN ($CD_3OD$) δ 0,87 (3H, s, 18-$CH_3$) ; 1,38 (3H, s, 19-$CH_3$); 2,8, 3,5 (2H chacun, t: J=7 Hz chacun, $CH_2$-$CH_2$-histamine); 4,2-4,7 (4H, q: J=19 Hz, 21-$CH_2O$) ; 4,4 (1H, m, 11α-H); 4,4 (2H, s, NOC$H_2$CO); 6,4 (1H, s, 4-H); 6,8, 7,6 (1H chacun, s chacun, CH-imidazole).

**Stade B - Traceur cortisol-3-CMO (syn)-[125I]iodohistamine**

Le marquage à l'iode 125 effectué en milieu liquide fait intervenir la chloramine T comme agent oxydant et le métabisulfite de sodium pour arrêter la réaction d'oxydation. Le protocole de iodation est le suivant : le précurseur de marquage (9) obtenu au stade A ci-dessus, 2 nanomoles environ, dilué dans 60 μl de tampon phosphate de sodium 0,2 M pH 8,0 est traité par 20 μl de la solution de l'iodure de sodium radioactif (1 nmol Na125I) en présence de 20 μl de solution de chloramine T à 5 mg/ml dans du tampon phosphate de sodium 0,2 M pH 8,0.

On laisse incuber 2 minutes à température ambiante puis arrête la réaction par addition de 100 μl de solution aqueuse de métabisulfite de sodium à 1 mg/ml. Le mélange réactionnel est dilué dans 700 μl d'eau, puis chromatographié sur colonne C18 μBondapak (phase inverse) selon les conditions d'élution suivantes :

| Temps (min) | Débit (ml/min) | Pompe A (eau) | Pompe B (acétonitrile) |
|---|---|---|---|
| Initial | 1 | 90 % | 10 % |
| 5 | 1 | 90 % | 10 % |
| 80 | 1 | 50 % | 50 % |

Dans ces conditions d'élution, le traceur cortisol-3-CMO (syn)-[125I]iodohistamine est élué à 49 min (37 % acétonitrile).

EXEMPLE 5 : Précurseur de marquage isomère (anti) du cortisol-3-CMO couplé à l'histamine (10) et traceur iodé radioactif correspondant.

**Stade A : Isomère (anti) du cortisol-3-CMO couplé à l'histamine (10).**

L'isomère (anti) du cortisol-3-CMO activé sous forme d'ester de N-hydroxysuccinimide (8) obtenu au stade A de l'exemple 3, est traité comme décrit ci-dessus dans le cas de l'isomère syn pour conduire au composé attendu: cortisol-3-CMO (anti)-histamine (10).
$t_r$ (anti) = 23,6 minutes (conditions de CLHP mentionnées ci-dessus)
$R_f$ = 0,25 (chloroforme-méthanol-ammoniaque 60:10:2)
F = 134-138°C (produit brut séparé par CLHP)
UV : $\lambda_{max}$ = 247 nm; ε= 23000 $M^{-1}cm^{-1}$
$^1$H-RMN ($CD_3OD$) δ 0,87 (3H, s, 18-$CH_3$) ; 1,35 (3H, s, 19-$CH_3$); 2,8, 3,5 (2H chacun, t: J=7 Hz chacun, $CH_2$-$CH_2$-histamine) ; 4,2-4,7 (4H, q: J=19 Hz, 21-$CH_2O$) ; 4,4 (1H, m, 11α-H); 4,4 (2H, s, $NOCH_2CO$); 5,6 (1H, s, 4-H); 6,8, 7,6 (1H chacun, s chacun, CH-imidazole).

**Stade B : Traceur cortisol-3-CMO (anti)-[125I]iodohistamine.**

On opère comme indiqué au stade B de l'exemple 4. Le traceur cortisol-3-CMO (anti) - [125I] iodohistamine est élué à 46 min (35,6 % acétonitrile).

EXEMPLE 6 : Anticorps induits par l'immunogène cortisol-3-CMO (syn)-SAB.

**Stade A - Immunisation des souris.**

Des souris Balb/c mâles, âgées de 6 semaines sont immunisées selon le programme suivant :

- Jour 0 : Injection par voie intrapéritonéale de 50 μg de cortisol-3-CMO (syn)-SAB dans une émulsion 50/50 (100 μl NaCl / 100 μl adjuvant de Freund complet).
- Jour 21 : Rappel par voie intrapéritonéale avec 50 μg de cortisol-3-CMO (syn)-SAB dans une émulsion 50/50 (100 μl NaCl / 100 μl adjuvant de Freund incomplet).
- Jour 42 : Rappel par voie intrapéritonéale avec 50 μg de cortisol-3-CMO (syn)-SAB dans une émulsion 50/50 (100 μl NaCl / 100 μl adjuvant de Freund incomplet).
- Jour 63 : Rappel par voie intrapéritonéale avec 50 μg de cortisol-3-CMO (syn)-SAB dans une émulsion 50/50 (100 μl NaCl / 100 μl adjuvant de Freund incomplet).
- Jour 84 : Rappel par voie intrapéritonéale avec 50 μg de cortisol-3-CMO (syn)-SAB dans une émulsion 50/50 (100 μl NaCl / 100 μl adjuvant de Freund incomplet).
- Jour 244 : Injection par voie intraveineuse de 100 μg de cortisol-3-CMO (syn)-SAB dans 100 μl de NaCl et une heure après, rappel par voie intrapéritonéale avec 300 μg de cet immunogène dans 300 μl de NaCl.
- Jour 247 : Fusion cellulaire.

**Stade B - Fusion cellulaire selon le protocole de Köhler et Milstein.**

a) Au jour 247, la souris sélectionnée est sacrifiée et sa rate prélevée et dilacérée. Les cellules spléniques sont lavées dans du milieu RPMI 1640. Les cellules de myélome P3.X63.Ag8 653, préalablement cultivées en RPMI 20% sérum de veau foetal (SVF), 1% glutamine, 1% acides aminés non essentiels et 1% pyruvate de sodium sont aussi lavées dans le même milieu.

Parallèlement, des macrophages péritonéaux sont obtenus par lavage du péritoine de souris Balb/c non immunisées avec du RPMI.

Pour obtenir la formation d'hybridomes, les splénocytes et les cellules de myélome sont mélangées dans un tube à raison de 5 cellules de rate pour 1 cellule de myélome. Après centrifugation, le culot cellulaire est remis en suspension dans 800 µl de polyéthylène glycol 1500 à 50% en tampon Hepes 75 mM pH 7,5. Après un contact de 1 minute à 37°C, 20 ml de milieu RPMI 1640 sont rajoutés lentement aux cellules fusionnées.

b) La culture initiale est réalisée sur plaques de microculture 96 puits en présence de milieu RPMI contenant 20% de sérum de veau foetal (SVF) et les additifs suivants : hypoxanthine $5\times10^{-3}$M, aminoptérine $2\times10^{-5}$ M et thymidine $8\times10^{-4}$ M. Dans chaque puits sont ajoutés $5\times10^3$ macrophages péritonéaux puis $10^5$ cellules fusionnées.

**Stade C - Clonages et sous-clonages.**

Chaque hybridome sélectionné suivant la technique citée au stade D ci-après, résulte d'un clonage par une technique en dilution limite dans laquelle 10, 5, 2, 1 et 0,5 cellules sont réparties statistiquement dans des micropuits contenant des macrophages péritonéaux. Deux sous-clonages sont ainsi réalisés, chaque clone et sous-clone ayant été répliqué puis congelé dans 90% de SVF et 10% de diméthylsulfoxyde (DMSO). Les sous-clones de dernière génération font enfin l'objet d'une expansion in vivo pour l'obtention de liquide d'ascite dans des souris Balb/c, suivie d'une purification des immunoglobulines sur protéine A.

**Stade D - Technique de sélection des cellules hybrides.**

La sélection est effectuée par méthode radio-immunologique en phase liquide à partir du surnageant de culture. Les surnageants contenant éventuellement des anticorps anti-cortisol sont incubés avec une solution de cortisol radioactif (cortisol tritié ou dérivé de cortisol marqué à l'iode radioactif: cortisol-3-CMO (anti) - [$^{125}$I] iodohistamine). S'il y a présence d'anticorps anti-cortisol, ceux ci vont lier le stéroïde radioactif. Après incubation avec du charbon-dextran et centrifugation pour éliminer la radioactivité libre et toutes les petites molécules non fixées aux anticorps, le surnageant (fraction liée) est prélevé et compté .

| Protocole de sélection | | | | | |
|---|---|---|---|---|---|
| Tubes | Surnageant de culture | Milieu RPMI +10 % SVF | Immunsérum de souris[1] | Traceur[2] | Tampon[3] |
| Essais | 70 µl | | | 100 µl | |
| Témoin négatif | | 70 µl | | 100 µl | |
| Témoin positif | | | 70 µl | 100 µl | |
| Totaux | | 70 µl | | 100 µl | 500 µl |
| Incubation durant 16 heures à 4° C | | | | | |
| Précipitation avec 500 µl de suspension de charbon[4] dextran durant 15 minutes à 4° C sauf dans les tubes "Totaux" | | | | | |
| Centrifugation 3 500 rpm pendant 15 minutes à 4° C | | | | | |
| Comptage de la radioactivité sur un prélèvement de 550 µl de surnageant:<br>- dans le cas du traceur tritié, le comptage s'effectue dans un compteur Bêta, après rajout de 6 ml de liquide scintillant.<br>- dans le cas du traceur iodé, le comptage se fait directement dans un compteur Gamma.. | | | | | |

[1] Immunsérum de souris induit par l'immunogène cortisol-3-CMO (syn)-SAB dilué 4000 fois dans du tampon phosphate de sodium 0,1 M, gélatine 0,1%, azoture de sodium 10 mM pH 7,2.

[2] Traceur [1,2,6,7-$^3$H]cortisol (50-80 Ci/mmol) à 10 000 cpm/100 µl de tampon phosphate de sodium 0,1 M, gélatine 0,1%, azoture de sodium 10 mM pH 7,2.

[2] Traceur cortisol-3-CMO (anti)-[$^{125}$I]iodohistamine à 35 000 cpm/100 µl de tampon phosphate de sodium 0,1 M, gélatine 0,1%, azoture de sodium 10 mM pH 7,2.

[3] Tampon = phosphate de sodium 0,1 M, gélatine 0,1%, azoture de sodium 10 mM pH 7,2.

[4] Suspension de charbon-dextran à 0,3g - 0,03g/100 ml de tampon phosphate de sodium 0,1 M, gélatine 0,1%, azoture de sodium 10 mM pH 7,2.

Seules sont retenues lors de chaque clonage, les cellules sécrétant les anticorps anti-cortisol reconnaissant à la fois le traceur cortisol tritié et le traceur cortisol-3-CMO (anti)-[125I]iodohistamine, avec un seuil de détection supérieur à 5 fois le témoin négatif.

La mesure des affinités des anticorps monoclonaux est effectuée par déplacement du traceur cortisol-3-CMO (anti)-[125I]iodohistamine avec les deux précurseurs de marquage : cortisol-3-CMO(anti)-histamine et cortisol-3-CMO (syn)-histamine. L'affinité est estimée (sous forme de valeur $CI_{50}$) d'après la concentration déplaçant la moitié du traceur, puisque celui-ci est en faible quantité vis-à-vis des autres constituants.

Le tableau ci-dessous indique les valeurs $CI_{50}$ de deux anticorps monoclonaux obtenus contre l'immunogène cortisol-3-CMO (syn)-SAB, pour lesquels on observe un déplacement plus important du traceur cortisol-3-CMO (anti)-[125I] iodohistamine par l'isomère syn que par l'isomère anti.

| Anticorps monoclonal | Déplacement du traceur par | | Rapport CI 50 Anti/ CI 50 Syn |
|---|---|---|---|
| | Cortisol-3-CMO (anti)-histamine CI 50 (ng/ml) | Cortisol-3-CMO (syn)-histamine CI 50 (ng/ml) | |
| Immu 473 | 0,496 | 0,182 | 2,73 |
| Immu 482 | 0,468 | 0,176 | 2,66 |

On a ainsi sélectionné l'anticorps IMMU-473 qui a été déposé à la Collection Nationale de Cultures de Microorganismes (CNCM) à Paris le 9 Février 1995 sous le numéro I-1532.

EXEMPLE 7 - Trousse de dosage direct du cortisol dans les sérums ou plasmas.

Le procédé cité est un dosage radio-immunologique par compétition dont le principe est le suivant : les sérums ou plasmas à doser ou les standards sont incubés dans des tubes recouverts d'anticorps monoclonaux anti-cortisol selon l'invention, avec l'un ou l'autre des deux traceurs cortisol-3-CMO (syn) - [125I] iodohistamine ou cortisol-3-CMO (anti) - [125I] iodohistamine. Après incubation à 22°C, le contenu des tubes est vidé par aspiration. La radioactivité liée est mesurée dans un compteur Gamma. Une courbe d'étalonnage est établie. Les valeurs inconnues des sérums ou plasmas sont déterminées par interpolation à l'aide de cette courbe.

Les réactifs et le protocole employés pour doser directement le cortisol dans les sérums et plasmas sont :

Réactifs :

Gamme standard cortisol en sérum humain débarrassé des stéroïdes et azoture de sodium à 20 mM, pH 7,2, établie pour 6 concentrations: 0; 20; 70; 200; 700 et 2000 nM (0; 7,25; 25,37; 72,5; 253,75 et 725 ng/ml).

Traceur cortisol-3-CMO (syn) - [125I] iodohistamine ou cortisol-3-CMO (anti) -[125I] iodohistamine des exemples 4 et 5 ajusté à 150 000 cpm / ml de tampon phosphate de sodium 0,1 M, gélatine 0,1%, azoture de sodium 10 mM et danazol 3,56 µM pH 7,2.

Tubes recouverts d'anticorps monoclonaux anti-cortisol IMMU-473 selon l'invention. La technique de greffage de ces anticorps sur phase solide est celle décrite dans FR-A-2.543.972.

Essai sur 87 sérums de patients.

Protocole :

Dans des tubes recouverts d'anticorps monoclonaux selon l'invention, sont déposés 25 µl de standard cortisol en sérum humain débarrassé des stéroïdes ou 25 µl d'échantillon biologique à doser et 0,5 ml de traceur cortisol radiomarqué. Après une heure d'incubation à 22°C avec agitation (400 rpm), le contenu des tubes est vidé et la radioactivité liée, immobilisée dans le tube, est mesurée dans un compteur Gamma.

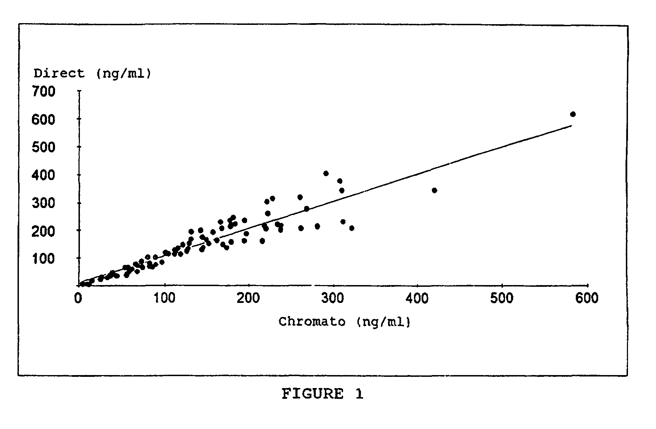

Ces dosages directs du cortisol selon l'invention ont été comparés aux dosages effectués après chromatographie des mêmes sérums sur des cartouches SEP PAK (Waters). La figure 1 représente la corrélation entre les deux techniques de dosage évoquées ci-dessus. En abscisse sont indiquées les valeurs obtenues par dosage après chromatographie et en ordonnée celles obtenues par dosage direct, toutes deux exprimées en ng/ml. Les résultats montrent une parfaite corrélation entre ces dosages directs et ceux effectués après chromatographie (nombre de sérums testés = 87).

La corrélation est la suivante :

$$Y_{(direct)} = 0,98\ X_{(chromatographie)} + 11,39$$

$$r = 0,92$$

où Y représente la valeur obtenue par dosage direct, X représente la valeur obtenue par dosage après chromatographie ; r est le coefficient de corrélation.

EXEMPLE 8 - Trousse de dosage direct du cortisol dans les urines.

Il s'agit également d'une technique de dosage radio-immunologique par compétition dont le principe est déjà décrit dans l'exemple 7. Dans ce procédé, on a utilisé les mêmes tubes recouverts d'anticorps monoclonaux IMMU-473 et le même traceur, indifféremment cortisol-3-CMO (syn)-[125I] iodohistamine ou cortisol-3-CMO (anti) - [125I] iodohistamine, que ceux du protocole de dosage direct des sérums. Seule, la gamme standard diffère : elle est en tampon avec une faible quantité de sérum-albumine bovine (voir ci-après).
Les réactifs et le protocole appliqués dans le dosage direct des urines sont les suivants :

Réactifs :

Gamme standard cortisol en tampon phosphate de sodium 0,1 M, SAB 3g/l, azoture de sodium 20 mM pH 7,2, établie pour 6 concentrations : 0; 20; 70; 200; 700 et 2000 nM (0; 7,25; 25,37; 72,5; 253,75 et 725 ng/ml).
Traceur cortisol-3-CMO (syn) - [125I] iodohistamine ou cortisol-3-CMO (anti)-[125I]iodohistamine ajusté à 150 000 cpm/ml de tampon phosphate de sodium 0,1 M, gélatine 0,1 %, danazol 3,56 μM, azoture de sodium 10 mM pH 7,2.
Tubes recouverts d'anticorps monoclonal anti cortisol IMMU-473 selon l'invention.

Essai sur 165 urines de patients.

Protocole :

50 μl de standard cortisol en tampon ou 50 μl d'urine à doser sont incubés dans des tubes revêtus d'anticorps monoclonal IMMU-473 avec 0,5 ml de traceur cortisol-3-CMO (syn) - [125I] iodohistamine ou cortisol-3-CMO (anti) - [125I] iodohistamine. Après une heure d'incubation à 22°C avec agitation (400 rpm), le contenu des tubes est vidé et la radioactivité liée, immobilisée dans le tube, est déterminée dans le compteur Gamma.
Nous avons comparé ces dosages directs avec ceux réalisés après chromatographie des urines sur des cartouches SEP PAK (Waters).

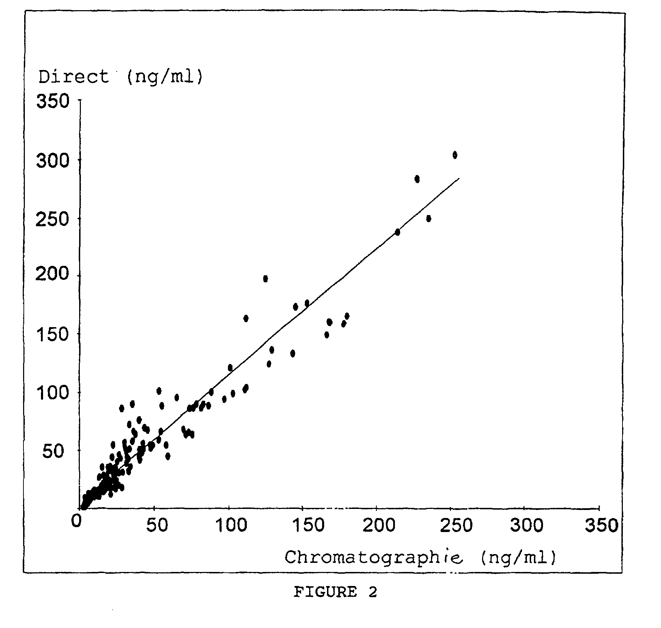

La figure 2 représente la corrélation entre les deux techniques de dosage évoquées ci-dessus pour les 165 urines. En abscisse sont indiquées les valeurs obtenues par dosage après chromatographie et en ordonnée celles obtenues par dosage direct, toutes deux exprimées en ng/ml. La corrélation est très bonne :

$$Y_{(direct)} = 1,09\ X_{(chromatographie)} + 5,3$$

$$r = 0,95$$

où Y représente la valeur obtenue par dosage direct, X représente la valeur obtenue par dosage après chromatographie et r est le coefficient de corrélation.
Le tableau 2 ci-après montre les résultats obtenus en utilisant deux procédés de dosage direct du cortisol urinaire de l'art antérieur comparés au procédé selon l'invention et au procédé de référence après chromatographie. Seul le procédé selon l'invention donne des résultats exacts; les deux autres procédés surestiment les concentrations des échantillons urinaires d'un facteur de 2 en moyenne.

TABLEAU 1

Diverses trousses de dosage du cortisol commercialisées

| FABRICANTS | KODAK | ORION | DSL | INCSTAR | RADIM | DIAGNOSTIC PASTEUR | DPC | CORNING | ICN | IMMUNOTECH 1ère génération | BIOMERICA | EUROGENETICS |
|---|---|---|---|---|---|---|---|---|---|---|---|---|
| Référence | IM-2021 | 68548 | DSL 2000 | CA-529 | KS 18 CT | 825 | TK COI | 472.360 MAGIC | 07.2211 02 | 1114 | 1019 | Cortisol Elisa |
| Techniq ue de dosage | RIA | RIA | RIA | RIA | RIA | RIA | RIA | RIA | RIA | RIA | EIA | EIA |
| Sérum | direct | direct | direct | direct | direct | direct | direct | direct | direct | direct | direct | direct |
| Urine | direct | direct ou extraction | direct ou extraction | extraction | extraction | extraction | extraction | extraction | extraction | extraction | extraction | extraction |

EP 0 733 903 B1

TABLEAU 2

| KITS \ URINES | CONCENTRATION CORTISOL (ng/ml) | | | |
|---|---|---|---|---|
| | ORION DIRECT | KODAK DIRECT | PRESENTE INVENTION | CHROMATOGRAPHIE |
| A | 27 | 19 | 10 | 8 |
| B | 9 | 10 | 8 | 8 |
| C | 23 | 24 | 15 | 11 |
| D | 48 | 35 | 18 | 19 |
| E | 38 | 42 | 20 | 21 |
| F | 62 | 66 | 36 | 33 |
| G | 63 | 55 | 34 | 33 |
| H | 85 | 73 | 40 | 45 |
| I | 110 | 84 | 45 | 48 |
| J | 101 | 84 | 47 | 59 |
| K | 175 | 105 | 56 | 64 |
| L | 417 | 253 | 158 | 166 |

Ces résultats montrent la bonne corrélation obtenue pour chaque urine testée entre le procédé de la présente invention et la technique de référence (chromatographie).

**Revendications**

**1.** Anticorps polyclonal ou monoclonal, reconnaissant spécifiquement le cortisol en milieu urinaire et dont le rapport

**EP 0 733 903 B1**

des affinités (CI50) de l'anticorps pour les dérivés cortisol-3-carboxyméthyloxime (syn)-histamine et cortisol-3-carboxyméthyloxime (anti)-histamine, est supérieur ou égal à 1 en faveur de l'isomère syn, tel qu'obtenu par utilisation d'un immunogène comprenant à titre de haptène le cortisol-3-carboxyméthyloxime (isomère syn) substantiellement pur.

2. Anticorps polyclonal ou monoclonal selon la revendication 1, **caractérisé en ce que** le rapport des affinités (CI50) de l'anticorps pour les dérivés cortisol-3-carboxyméthyloxime (syn)-histamine et cortisol-3-carboxyméthyloxime (anti)-histamine, est supérieur ou égal à 1,5 en faveur de l'isomère syn.

3. Anticorps polyclonal ou monoclonal selon la revendication 1 ou 2, **caractérisé en ce qu'**il est produit par immunisation d'animaux contre un composé immunogène constitué du haptène cortisol-3-carboxyméthyloxime (isomère syn) substantiellement pur, couplé par sa fonction carboxylique à une macromolécule.

4. Anticorps polyclonal ou monoclonal selon la revendication 3, **caractérisé en ce que** l'immunogène est constitué du haptène cortisol-3-carboxyméthyloxime (syn) couplé à la sérum albumine bovine avec un taux de couplage supérieur ou égal à 1.

5. Anticorps polyclonal ou monoclonal selon la revendication 4, **caractérisé en ce que** le taux de couplage est compris entre 15 et 31.

6. Anticorps polyclonal ou monoclonal selon l'une des revendications 1 à 5, **caractérisé en ce qu'**il reconnaît les composés cortisol-3-carboxyméthyloxime (syn)-histamine et cortisol-3-carboxyméthyloxime (anti)-histamine iodés, avec la même affinité.

7. Un anticorps produit par l'hybridome IMMU-473 déposé le 9 Février 1995 à la Collection Nationale des Cultures de Microorganismes sous le numéro I-1532.

8. Procédé de dosage immunologique du cortisol **caractérisé en ce que** l'on utilise un anticorps tel que défini à l'une des revendications 1 à 7, **en ce que** le dosage est un dosage par compétition utilisant un traceur et que le traceur est constitué du haptène cortisol-3-carboxyméthyloxime (syn) ou cortisol-3-carboxyméthyloxime (anti) couplé par sa fonction carboxylique à un marqueur qui est une molécule iodable, ou encore que le traceur est le cortisol tritié ou un dérivé tritié du cortisol .

9. Procédé de dosage immunologique du cortisol selon la revendication 8, **caractérisé en ce que**, à titre de traceur, cortisol-3-carboxyméthyloxime (syn) ou le cortisol-3-carboxyméthyloxime (anti) est couplé à l'histamine iodée radioactive.

10. Procédé de dosage immunologique du cortisol selon la revendication 8 ou 9, **caractérisé en ce que** le traceur est iodé à l'iode 125.

11. Procédé de dosage immunologique du cortisol **caractérisé en ce que** l'on utilise un anticorps tel que défini à l'une des revendications 1 à 7 et que le cortisol à doser est mis en compétition avec un traceur qui est un conjugué constitué de cortisol-3-carboxyméthyloxime (syn) ou cortisol-3-carboxyméthyloxime (anti) couplé à une enzyme ou à une molécule luminescente ou fluorescente.

12. Procédé de dosage immunologique du cortisol mettant en oeuvre le cortisol-3-carboxyméthyloxime (syn) ou le cortisol-3-carboxyméthyloxime (anti) fixé sur phase solide et un anticorps anti-cortisol tel que défini à l'une des revendications 1 à 7, marqué par une molécule radioactive, enzymatique, luminescente ou fluorescente.

13. Procédé de dosage immunologique du cortisol selon l'une des revendications 8 à 12, **caractérisé en ce que** l'anticorps est un anticorps monoclonal.

14. Trousse de dosage du cortisol dans les fluides biologiques utilisant un anticorps tel que défini à l'une des revendications 1 à 7.

15. Le cortisol-3-carboxyméthyloxime (syn), sous forme substantiellement pure.

16. Le cortisol-3-carboxyméthyloxime (syn)-histamine, sous forme substantiellement pure.

**17.** Le cortisol-3-carboxyméthyloxime (syn) couplé à une macromolécule, sous forme substantiellement pure.

**Patentansprüche**

**1.** Polyklonaler oder monoklonaler Antikörper, der Cortisol in Harnmilieu spezifisch erkennt und bei dem das Affinitätsverhältnis (CI50) des Antikörpers für die Derivate Cortisol-3-carboxymethyloxim-(syn)-histamin und Cortisol-3-carboxymethyloxim-(anti)-histamin größer oder gleich 1 zugunsten des syn-Isomers ist, wie es unter Verwendung eines Immunogens umfassend im Wesentlichen reines Cortisol-3-carboxymethyloxim (syn-Isomer) als Hapten erhalten wird.

**2.** Polyklonaler oder monoklonaler Antikörper nach Anspruch 1, **dadurch gekennzeichnet, dass** das Affinitätsverhältnis (CI50) des Antikörpers für die Derivate Cortisol-3-carboxymethyloxim-(syn)-histamin und Cortisol-3-carboxymethyloxim-(anti)-histamin größer oder gleich 1,5 zugunsten des syn-Isomers ist.

**3.** Polyklonaler oder monoklonaler Antikörper nach Anspruch 1 oder 2, **dadurch gekennzeichnet, dass** er durch die Immunisierung von Tieren gegen eine immunogene Verbindung bestehend aus dem im Wesentlichen reinen Hapten Cortisol-3-carboxymethyloxim (syn-Isomer), das über seine Carboxylfunktion an ein Makromolekül gekoppelt ist, erzeugt wird.

**4.** Polyklonaler oder monoklonaler Antikörper nach Anspruch 3, **dadurch gekennzeichnet, dass** das Immunogen aus dem Hapten 3-Cortisol-carboxymethyloxim (syn) besteht, gekoppelt an Rinderserumalbumin mit einem Kopplungsgrad von 1 oder mehr.

**5.** Polyklonaler oder monoklonaler Antikörper nach Anspruch 4, **dadurch gekennzeichnet, dass** der Kopplungsgrad zwischen 15 und 31 beträgt.

**6.** Polyklonaler oder monoklonaler Antikörper nach einem der Ansprüche 1 bis 5, **dadurch gekennzeichnet, dass** er die iodierten Verbindungen Cortisol-3-carboxymethyloxim-(syn)-histamin und Cortisol-3-carboxymethyloxim-(anti)-histamin mit derselben Affinität erkennt.

**7.** Antikörper, produziert durch das Hybridom IMMU-473, das am 9. Februar 1995 bei der Collection Nationale des Cultures de Microorganismes unter der Nummer I-1532 hinterlegt wurde.

**8.** Verfahren zur immunologischen Bestimmung von Cortisol, **dadurch gekennzeichnet, dass** ein Antikörper nach einem der Ansprüche 1 bis 7 verwendet wird, dass die Bestimmung eine kompetitive Bestimmung unter Verwendung eines Tracers ist und dass der Tracer aus dem Hapten Cortisol-3-carboxymethyloxim (syn) oder Cortisol-3-carboxymethyloxim (anti), das über seine Carboxylfunktion an eine Markersubstanz in Form eines iodierbaren Moleküls gebunden ist, besteht, oder aber der Tracer tritiummarkiertes Cortisol oder ein tritiummarkiertes Derivat von Cortisol ist.

**9.** Verfahren zur immunologischen Bestimmung von Cortisol nach Anspruch 8, **dadurch gekennzeichnet, dass** Cortisol-3-carboxymethyloxim (syn) oder Cortisol-3-carboxymethyloxim (anti) als Tracer an radioaktives iodiertes Histamin gebunden ist

**10.** Verfabren zur immunologischen Bestimmung von Cortisol nach Anspruch 8 oder 9, **dadurch gekennzeichnet, dass** der Tracer mit Iod 125 iodiert ist.

**11.** Verfahren zur immunologischen Bestimmung von Cortisol, **dadurch gekennzeichnet, dass** ein Antikörper nach einem der Ansprüche 1 bis 7 verwendet wird und dass das zu bestimmende Cortisol in Konkurrenz mit einem Tracer in Form eines Konjugats gestellt wird, das aus Cortisol-3-carboxymethyloxim (syn) oder Cortisol-3-carboxymethyloxim (anti), gekoppelt an ein Enzym oder ein lumineszicrendes oder fluoreszierendes Molekül, besteht.

**12.** Verfahren zur immunologischen Bestimmung von Cortisol, bei dem an feste Phase gebundenes Cortisol-3-carboxymethyloxim (syn) oder Cortisol-3-carboxymethyloxim (anti) und ein Anti-Cortisol-Antikörper nach einem der Ansprüche 1 bis 7 verwendet werden, der mit einem radioaktiven, enzymatischen, lumineszierenden oder fluoreszierenden Molekül markiert ist.

**13.** Verfahren zur immunologischen Bestimmung von Cortisol nach einem der Ansprüche 8 bis 12, **dadurch gekennzeichnet, dass** der Antikörper ein monoklonaler Antikörper ist.

**14.** Set zur Bestimmung von Cortisol in biologischen Flüssigkeiten unter Verwendung eines Antikörpers nach einem der Ansprüche 1 bis 7.

**15.** Cortisol-3-carboxymethyloxim-(syn) in im Wesentlichen reiner Form.

**16.** Cortisol-3-carboxymethyloxim-(syn)-histamin in im Wesentlichen reiner Form.

**17.** Cortisol-3-carboxymethyloxim (syn), gekoppelt an ein Makromolekül, in im Wesentlichen reiner Form.

**Claims**

**1.** A polyclonal or monoclonal antibody recognizing specifically cortisol in a urine medium, and of which the ratio of affinities (IC50) of the antibody for the derivatives cortisol-3-carboxymethyloxime (syn)-histamine and cortisol-3-carboxymethyloxime (anti)-histamine is greater than or equal to 1 in favour of the syn isomer, as obtained by using an immunogen comprising substantially pure cortisol-3-carboxymethyloxime (syn isomer) as hapten.

**2.** A polyclonal or monoclonal antibody according to claim 1, **characterized in that** the ratio of affinities (IC50) of the antibody for the derivatives cortisol-3-carboxymethyloxime (syn)-histamine and cortisol-3-carboxymethyloxime (anti)-histamine is greater than or equal to 1.5 in favour of the syn isomer.

**3.** A polyclonal or monoclonal antibody according to claim 1 or 2, **characterized in that** it is produced by immunisation of animals against an immunogen compound composed of the substantially pure hapten cortisol-3-carboxymethyloxime (syn isomer) coupled by its carboxyl function to a macromolecule.

**4.** A polyclonal or monoclonal antibody according to claim 3, **characterized in that** the immunogen is composed of the hapten cortisol-3-carboxymethyloxime (syn) coupled to bovine serum albumin with a coupling ratio greater than or equal to 1.

**5.** A polyclonal or monoclonal antibody according to claim 4, **characterized in that** the coupling ratio is between 15 and 31.

**6.** A polyclonal or monoclonal antibody according to one of claims 1 to 5, **characterized in that** it recognises the iodinated cortisol-3-carboxymethyloxime (syn)-histamine and cortisol-3-carboxymethyloxime (anti)-histamine compounds with the same affinity.

**7.** An antibody produced by the hybridoma IMMU-473 filed on 9 February 1995 with the Collection Nationale des Cultures de Microorganismes under number I-1532.

**8.** An immunoassay process for cortisol, **characterized in that** an antibody as defined in one of claims 1 to 7 is used, **in that** the assay is a competitive assay using a tracer and that the tracer is composed of the hapten cortisol-3-carboxymethyloxime (syn) or cortisol-3-carboxymethyloxime (anti) coupled by its carboxyl function to a label which is a molecule capable of being iodinated, or also that the tracer is tritiated cortisol or a tritiated derivative of cortisol.

**9.** An immunoassay process for cortisol according to claim 8, **characterized in that**, by way of a tracer, cortisol-3-carboxymethyloxime (syn) or cortisol-3-carboxymethyloxime (anti) is coupled to radioactive iodinated histamine.

**10.** An immunoassay process for cortisol according to claim 8 or 9, **characterized in that** the tracer is iodinated with iodine-125.

**11.** An immunoassay process for cortisol, **characterized in that** an antibody as defined in one of claims 1 to 7 is used, and that the cortisol to be assayed competes with a tracer which is a conjugate composed of cortisol-3-carboxymethyloxime (syn) or cortisol-3-carboxymethyloxime (anti) coupled to an enzyme or to a luminescent or fluorescent molecule.

**12.** An immunoassay process for cortisol employing cortisol-3-carboxymethyloxime (syn) or cortisol-3-carboxymethyloxime (anti) fixed to a solid phase and an anti-cortisol antibody as defined in one of claims 1 to 7, labelled by a radioactive, enzyme, luminescent or fluorescent molecule.

**13.** An immunoassay process for cortisol according to one of claims 8 to 12, **characterized in that** the antibody is a monoclonal antibody.

**14.** An assay kit for cortisol in biological fluids using an antibody as defined in one of claims 1 to 7.

**15.** Cortisol-3-carboxymethyloxime (syn), in the substantially pure form.

**16.** Cortisol-3-carboxymethyloxime (syn)-histamine in the substantially pure form.

**17.** Cortisol-3-carboxymethyloxime (syn) coupled to a macromolecule, in the substantially pure form.

FIGURE 1

FIGURE 2